# EUROPEAN PATENT APPLICATION

(11) **EP 4 785 867 A1**
(43) Date of publication of application: **05.08.2026**
(21) Application number: 25155621.3
(22) Date of filing: 03.02.2025
(51) Int. Cl.: A61B 6/00, G01R 33/54

(54) **PARAMETER SETTING FOR MEDICAL IMAGING PROCEDURES**

(71) Applicant: Koninklijke Philips N.V., 5656 AE Eindhoven (NL)
(72) Inventor: WIEMKER, Rafael, Eindhoven (NL); DENISSEN, Adrianus Johannes Maria, Eindhoven (NL); VAN EE, Raymond, 5656AG Eindhoven (NL); KRUEGER, Sascha, Eindhoven (NL); HARDER, Tim Philipp, Eindhoven (NL); BUELOW, Thomas, Eindhoven (NL); SENEGAS, Julien Thomas, Eindhoven (NL); WIRTZ, Daniel, Eindhoven (NL)
(74) Representative: Philips Intellectual Property & Standards

(57) **Abstract**

Disclosed is a method (300) for parameter setting for a medical imaging procedure. The method comprises: controlling (S1) a motorized patient support (120) to apply an excitation to patient body tissue; monitoring (S2) a response of the patient body tissue to the applied excitation; determining (S3) composition of the patient body tissue based on the monitored response; and determining (S4) values of one or more parameters for use in conjunction with the medical imaging procedure based on the determined composition. The method facilitates resource efficient body composition estimation for parameter setting during medical imaging procedures.

## Description

### FIELD OF THE INVENTION

The invention relates to systems and methods for parameter setting for medical imaging procedures.

### BACKGROUND OF THE INVENTION

The amount of radiation required for a computed tomography (CT) scan can depend on the patient's body composition. Fat tissue absorbs less radiation than denser tissues like muscle and bone. Understanding the distribution of fat and fat-free mass can facilitate calculation of the radiation dose, ensuring sufficient image quality while reducing exposure. Various techniques for estimating body composition are known in the art. One example of such a technique is elastography, which is typically performed by using a dedicated point source to apply vibration to an isolated region of body tissue, and recording the response during an magnetic resonance (MR), ultrasound (US), or CT scan. Further examples include bioelectrical impedance analysis (BIA) and electrical impedance tomography (EIT), which estimate body composition by measuring resistance of body tissues to oscillating electrical currents. Dual-energy X-ray absorptiometry (DEXA) is considered the gold standard for body composition estimation. It uses two low-dose X-ray beams to differentiate between bone mass, fat mass, and fat-free mass. All of these techniques introduce additional equipment, preparation time and cost to the workflow and some of them involve increased radiation exposure.

### SUMMARY OF THE INVENTION

It would be desirable to estimate body composition for parameter setting during medical imaging procedures in a resource efficient way, that is, with minimal or at least reduced impact on equipment requirements and workflow.

To better address one or more of these concerns, there is provided, in a first aspect of the invention, a method for parameter setting for a medical imaging procedure. The method comprises: controlling a motorized patient support to apply an excitation to patient body tissue; monitoring a response of the patient body tissue to the applied excitation; determining composition of the patient body tissue based on the monitored response; and determining values of one or more parameters for use in conjunction with the medical imaging procedure based on the determined composition.

### Applying the excitation

Controlling the motorized patient support may comprise causing the patient support to vibrate in at least one dimension to elicit the excitation. The excitation applied to the patient body tissue may comprise a predetermined excitation pattern. The predetermined excitation pattern may be at least two dimensional, to facilitate its recognition in the monitored response. For example, the predetermined excitation pattern may comprise one or more circles or ellipses. For expediency, the excitation may be applied during movement of the patient body tissue into position for the medical imaging procedure. For example, an acceleration function which causes the patient support to move the patient body tissue into position for the medical imaging procedure may be adapted to comprise one or more features to apply the excitation to the patient body tissue. The one or more features may comprise a delta in acceleration or deacceleration. To mitigate disruption to the patient resulting from the excitation, the excitation may exhibit one or more of: a shorter duration; a higher frequency; and a smaller amplitude than positioning movements, that is, movements for moving the patient body tissue into position for the medical imaging procedure. In that regard, controlling the motorized patient support may further comprise causing the patient support to execute such positioning movements. The excitation applied to the patient body tissue may exhibit a single predetermined frequency or a range of frequencies. The applied excitation pattern may comprise a single predetermined shape or a range of shapes.

### Monitoring the response

The response of the patient body tissue to the applied excitation may be monitored globally. However, more preferably, monitoring the response of the patient body tissue to the applied excitation may comprise monitoring a plurality of local responses of the patient body tissue to the applied excitation. The response may be monitored in at least two dimensions, especially in the case that the applied excitation pattern is at least two dimensional. To reduce or minimize involvement of extraneous equipment, the response may be monitored using a pressure sensing mattress and/or a camera. Monitoring the response of the patient body tissue to the applied excitation may comprise monitoring for a single predetermined frequency of excitation or sweeping over a range of frequencies. Monitoring the response of the patient body tissue to the applied excitation may comprise monitoring for a single predetermined shape of the applied excitation pattern or sweeping over a range of shapes.

### Determining body composition

Determining the composition may comprise analyzing the monitored response to determine a body composition estimate. Determining the composition may comprise analyzing the plurality of monitored local responses (as described herein) to determine a spatially resolved body composition estimate. The body composition estimate may comprise an estimate of one or more of: fat/fat-free mass composition; fat/muscle ratio; soft-fat versus hard-fat tissue. The body composition estimate may be MR-compatible. Analyzing the monitored response comprises distinguishing tissue types based on their characteristic response to the excitation. Analyzing the monitored response may comprise distinguishing tissue types using elastography. The monitored response may be analyzed using knowledge of the applied excitation. The monitored response may be analyzed using biophysical analysis. The monitored response may be analyzed using machine learning.

### Parameter setting

The determined parameters being set by the method may comprise one or more of: radiation dose; radiation dose distribution; contrast agent dose; local magnetic field strength; local magnetic field gradient; scan duration; radio frequency coil choice; radio frequency sequence choice.

### Medical imaging procedure

The method may further comprise carrying out the medical imaging procedure using the determined values of the one or more parameters. In particular, the method may further comprise using the determined values to program equipment for use during the medical imaging procedure. That equipment may comprise the medical imaging system which carries out the medical imaging procedure. The equipment may comprise a dose injector for injecting contrast agent into the patient. Instead of, or in addition to, their use in the automatic programming of equipment, the determined values may be output for display to a clinician. The output may comprise a set of suggested parameters for use during the medical imaging procedure.

In a second aspect, there is provided a method for diagnostic monitoring. The method comprises: controlling a motorized patient support to apply an excitation to patient body tissue; monitoring a response of the patient body tissue to the applied excitation; determining composition of the patient body tissue based on the monitored response; and diagnosing one or more conditions based on the determined composition. The conditions include one or more of: cardiovascular disease; diabetes; metabolic syndrome; Alzheimer's.

### Sensor apparatus and sensor nodes

In an example, the sensor apparatus is configured as a sensing mattress, and the sensor nodes are arranged as an array of sensor nodes contained within the sensing mattress. The sensor nodes of the array may be configured to measure one or more physical quantities, e.g., pressure, temperature, electrical field (EEG), magnetic field (MEG). The sensing mechanism for a particular physical quantity may comprise one or more of: fiber optical (advantageous for MR- and CT imaging compatibility); electrical; piezoelectrical; quantum-magnetic (e.g., SQUIDs); gas bellow sensors; capacitive. The sensor nodes of the array may be of the same kind, or of mixed type. In an example the sensing mattress comprises a pressure-sensing array (such as an optical waveguide pressure-sensing array), which comprises a plurality of intersecting optical fibers forming the array, with each intersection serving as a pressure sensor node. Numerous array configurations are conceivable, exhibiting various shapes and arrangements, such as rectangular or non-rectangular arrays, the latter comprising e.g. circular or hexagonal arrays.

### Medical imaging system

The medical imaging system may comprise any suitable system such as a magnetic resonance imaging (MRI) system, a computed tomography (CT) system, or a hybrid system such as PET-CT or PET-MR.

Described herein is utilization of a pressure sensing mattress, or an optical camera, to observe local tissue responses to known excitation patterns applied by a motorized scan table. Corresponding signal processing delivers an estimate of body composition, which is advantageously obtained before the scan, to facilitate improved automatic planning of scan radiation and contrast agent dosage. Preferably, the estimate comprise a spatially resolved distribution pattern of the fat/muscle ratio.

The method of the first and/or second aspect may be computer implemented.

According to a third aspect, there is provided a computing system configured to perform the method of the first and/or second aspect.

According to a fourth aspect, there is provided a computer program (product) comprising instructions which, when executed by a computing system, enable or cause the computing system to perform the method of the first and/or second aspect.

According to a fifth aspect, there is provided a computer-readable (storage) medium comprising instructions which, when executed by a computing system, enable or cause the computing system to perform the method of the first and/or second aspect. The computer-readable medium may be transitory or non-transitory, volatile or non-volatile.

The computing system can typically comprise a processor, for example a processor that is part of a computer.

The term "obtaining", as used herein, may encompass receiving from another system, device, or process; receiving via an interaction with a user; loading or retrieving from storage or memory; measuring or capturing using sensors or other data acquisition circuitry.

The term "determining", as used herein, may encompass calculating, computing, processing, deriving, investigating, resolving, selecting, choosing, ascertaining, establishing, looking up (e.g., looking up in a table, a database or another data structure), receiving (e.g., receiving information), accessing (e.g., accessing data in a memory), and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

The above-described aspects will become apparent from, and elucidated with, reference to the detailed description provided hereinafter.

### BRIEF DESCRIPTION OF THE DRAWINGS

A detailed description will now be given, by way of example only, with reference to the accompanying drawings, in which:
Fig. 1 illustrates a medical imaging procedure being carried out;
Fig. 2 is a flowchart illustrating a method for parameter setting for the medical imaging procedure illustrated in Fig. 1; and
Fig. 3 illustrates a computing system that can be used in accordance with the systems and methods disclosed herein.

### DETAILED DESCRIPTION OF EMBODIMENTS

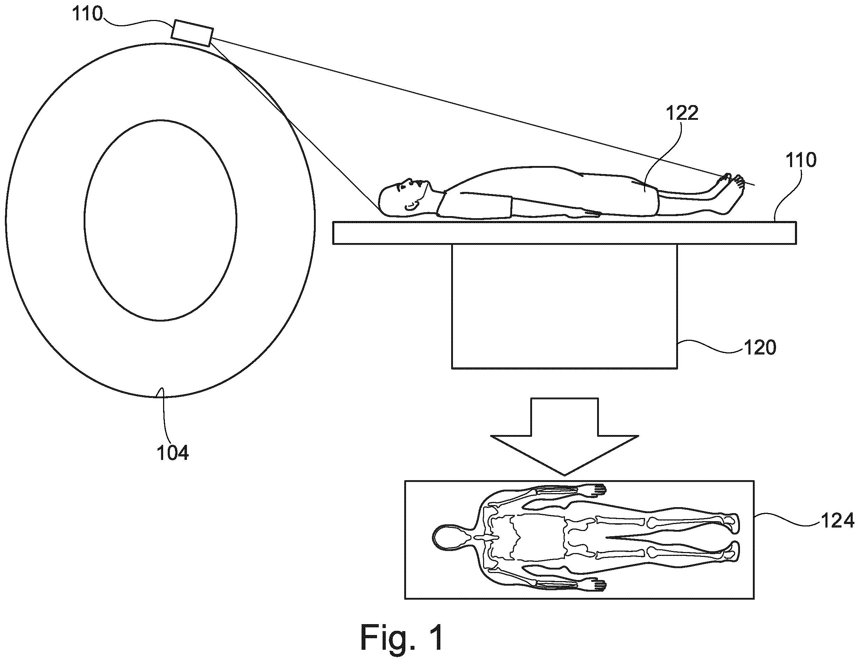

Fig. 1 illustrates a medical imaging procedure being carried out. A medical imaging system 104 (e.g. CT, MR) is configured to carry out the medical imaging procedure for generating an image volume depicting at least part of a patient 122. The medical imaging procedure may comprise a scan sequence for generating the image volume. The patient 122 is presented to the medical imaging system 104 by way of a motorized patient support 120, alternatively known as a scanner table, which is movable in at least one dimension by means of at least one motor provided for that purpose. In an example, the at least one motor comprises a step motor. A sensor apparatus 110 is arranged to obtain signals at a plurality of body locations of the patient 122. In one non-limiting example, the sensor apparatus 110 comprises an optical waveguide pressure sensing array such as that described in WO 2017/144675 A1, configured as a sensing mattress. The sensing mattress 110 comprises a plurality of sensor nodes arranged in a spatial configuration, such as an array. The spatial configuration may be rectangular, circular, or hexagonal, according to different applications and user needs. The sensor nodes can be embedded within or attached to the sensing mattress 110. In the latter case, the sensor nodes may be permanently or removably attached, with removability enabling flexibility in maintenance and replacement. The sensing mattress 110 is placed underneath the patient 122, that is, between the patient's body and the patient support 120. The sensing mattress 110 may be affixed to or integrated into the patient support 120. Instead of, or in addition to, the sensing mattress, the sensor apparatus 110 may comprise a camera 110 arranged to obtain signals at a plurality of body locations of the patient 122. The camera 110 may be an optical camera, for example in-bore or scanner-affixed.

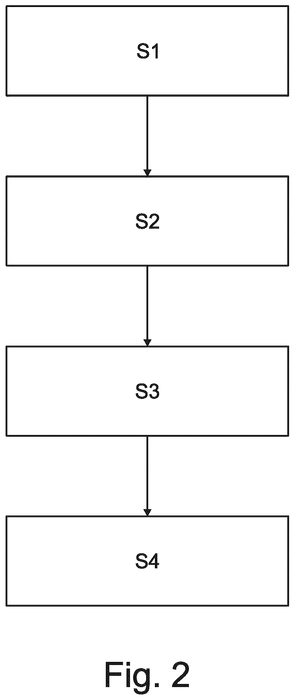

Fig. 2 is a flowchart illustrating a method for parameter setting for the medical imaging procedure illustrated in Fig. 1. The method comprises the following steps.

Excitation application step S1 comprises controlling the motorized patient support 120 to apply an excitation to patient body tissue. As described herein, the patient support 120 is motorized to facilitate motion, for example remote-controlled sliding motion, for moving the patient 122 into and out of the medical imaging system 104 and for putting the patient 122 into the proper position for imaging. According to the present disclosure, the same motor or motors which are used for moving the patient support 120 are further utilized for applying the excitation to patient tissue for determining body composition. The motorized patient support 120 is caused to vibrate in at least one of the x, y, z - directions (or any combination thereof, e.g. circles, ellipses) to excite minuscule vibration in patient tissue for a short period before the actual scan. Fat vs non-fat body mass, as well as soft-fat vs hard-fat tissue, can then be distinguished in later steps by virtue of distinct motion response patterns and amplitudes under vibration. It is conceivable that such table vibration using a short time window, high frequencies and small amplitudes may not cause severe inconvenience or irritation for the patient 122, particularly if announced in advance.

In one embodiment, the excitation (i.e. vibration of the patient support 120) uses a single predetermined (e.g., optimal) frequency, or a chirp, i.e. a decaying frequency to sample a certain range of frequencies.

In another embodiment, the acceleration function of the table is modified to contain features that excite body tissue vibration resonances, e.g. a short delta-peak of acceleration/deacceleration during the typical speed ramp-up or during the phase of constant table speed. This facilitates tissue analysis without significant changes in the workflow. Note, that some standard acceleration patterns natively contain broadband components already (e.g. multi-step acceleration patterns).

Response monitoring step S2 comprises monitoring a response of the patient body tissue to the applied excitation. The tissue response is monitored using the sensor apparatus 110, which monitors the magnitude and/or direction of the tissue response. The present disclosure foresees two alternative modes of operation which may be used in tandem or in isolation. In the first mode, the response of the body tissue to the applied vibration is observed using the sensing mattress 110. In the second mode, the response of the body tissue to the applied vibration is observed using the camera 110. The sensing mattress 110 yields direct univariate pressure time signal curves for each array point but constitutes an additional piece of equipment to be added to requirements. The camera-based approach has the advantage of not requiring any additional equipment. That is, the camera 110 already forms part of the medical imaging system 104, as a standard-speed in-bore camera (e.g. VitalEye), or as a replacement high-speed camera. The tissue response may be captured in the camera-based approach using interference techniques such as stroboscopic Fourier analysis. However, the camera-based approach may be feasible only for naked, uncovered body skin, and requires a preprocessing step converting the optical flow at each position into a local time signal, which may be uni- or bivariate (x-y).

Body composition determination step S3 comprises determining composition of the patient body tissue based on the monitored response. The monitored response or observed vibration is analyzed using knowledge of the applied excitation pattern (shape as well as frequency) using tissue elastography. Elastography assesses tissue elasticity, which is the tendency of tissue to resist deformation with an applied mechanical force, or to resume its original shape after removal of the force. Using the techniques described herein, measurements are acquired using the sensor apparatus 110 that can detect tissue stiffness in response to the mechanical force applied by the patient support 120 itself, not by a separate source. The excitation pattern may be applied globally over the scan table, but it elicits locally varying responses, since different kinds of tissue such as fat and muscle tissue have distinct elasticity modules and different resonance eigen-frequencies. Biophysical analysis or machine learning facilitates estimation of the fat/fat-free mass ratio for each location, so that a spatially resolved, material distribution map 104 of body composition can be generated, as illustrated in Fig. 1. In the case of machine learning, the model is trained using the observed excitation response together with the body composition derived from CT- or MR-acquired images as ground truth, that is, from patients with known body mass composition.

Parameter setting step S4 comprises determining values of one or more parameters for use in conjunction with the medical imaging procedure based on the determined composition. In the case of imaging, the medical imaging system 104 (e.g., a CT scanner) may use adaptive protocols that adjust the radiation dose based on the patient's body composition to achieve the best balance between image quality and safety. In the case of radiation therapy, an accurate body composition estimation may be used in radiation therapy planning, where different tissues absorb radiation differently, influencing dose distribution. In the case that a contrast agent is used, the dosage of injected contrast agent may take into account parameters such as fat and fat-free body mass. Optionally, the body composition information may be utilized to automatically program equipment involved in the medical imaging procedure, such as a dose injector for injecting the contrast agent, without any required intervention by a technologist.

In one variant to the method of Fig. 2, the method is instead performed for the purpose of diagnostic monitoring. Steps S1-S3 remain the same, but step S4 becomes diagnosis step S4 comprising diagnosing one or more conditions based on the determined body composition. The conditions may include one or more of: cardiovascular disease; diabetes; metabolic syndrome; Alzheimer's. The distribution of fat, especially visceral fat (fat around internal organs), is a significant indicator of health risks like cardiovascular disease, diabetes, and metabolic syndrome. The techniques described herein can quantify visceral fat and therefore assess the risk of these conditions.

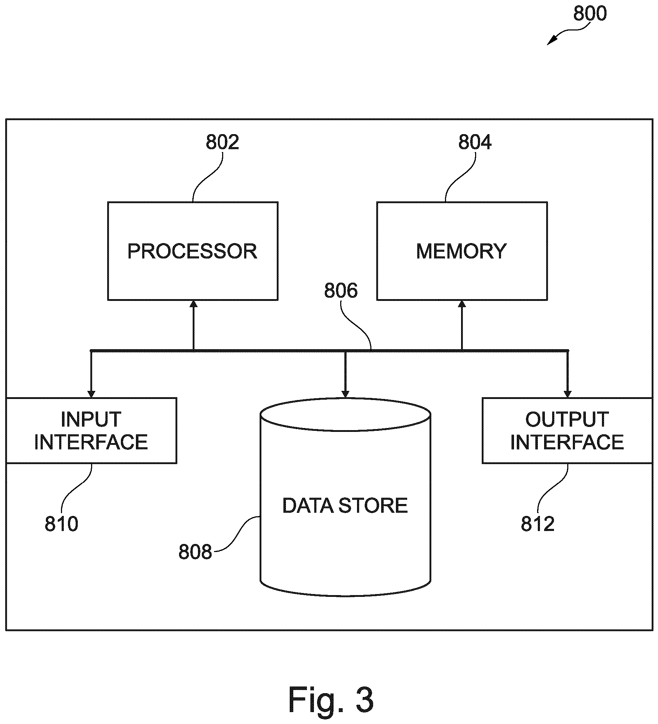

Fig. 3 illustrates an exemplary computing system 800 that can be used in accordance with the systems and methods disclosed herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by one or more components described herein or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

Alternatively, or in addition, the functionally described herein can be performed, at least in part, by one or more hardware logic components. For example, and without limitation, illustrative types of hardware logic components that can be used include field-programmable gate arrays (FPGAs), application-specific integrated circuits (ASICs), application-specific standard products (ASSPs), systemon-chip systems (SOCs), complex programmable logic devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. Method for parameter setting for a medical imaging procedure, the method comprising:
controlling a motorized patient support to apply an excitation to patient body tissue;
monitoring a response of the patient body tissue to the applied excitation;
determining composition of the patient body tissue based on the monitored response; and
determining values of one or more parameters for use in conjunction with the medical imaging procedure based on the determined composition.

2. The method as claimed in claim 1, wherein controlling the motorized patient support comprises causing the patient support to vibrate in at least one dimension to elicit the excitation.

3. The method as claimed in claim 1 or 2, wherein the excitation applied to the patient body tissue exhibits a predetermined excitation pattern.

4. The method as claimed in claim 3, wherein the predetermined excitation pattern is at least two dimensional.

5. The method as claimed in claim 3 or 4, wherein the predetermined excitation pattern comprises one or more circles or ellipses.

6. The method as claimed in any preceding claim, wherein the excitation is applied during movement of the patient body tissue into position for the medical imaging procedure.

7. The method as claimed in any preceding claim, wherein monitoring the response of the patient body tissue to the applied excitation comprises monitoring a plurality of local responses of the patient body tissue to the applied excitation.

8. The method as claimed in any preceding claim, wherein determining the composition of the patient body tissue comprises analyzing the monitored response to determine a body composition estimate.

9. The method as claimed in claims 7 and 8, wherein determining the composition comprises analyzing the plurality of monitored local responses to determine a spatially resolved body composition estimate.

10. The method as claimed in claim 8 or 9, wherein analyzing the monitored response comprises distinguishing tissue types based on their characteristic response to the excitation.

11. The method as claimed in any preceding claim, wherein the parameters comprise one or more of: radiation dose; radiation dose distribution; contrast agent dose; local magnetic field strength; local magnetic field gradient; scan duration; radio frequency coil choice; radio frequency sequence choice.

12. The method as claimed in any preceding claim, further comprising using the determined values to program equipment for use during the medical imaging procedure.

13. The method as claimed in any preceding claim, further comprising carrying out the medical imaging procedure using the determined values of the one or more parameters.

14. A computing system configured to perform the method of any preceding claim.

15. A computer-readable medium comprising instructions which, when executed by a computing system, cause the computing system to perform the method of any of claims 1-13.
